# EUROPEAN PATENT SPECIFICATION

(11) **EP 3 222 303 B1**
(45) Date of publication and mention of the grant of the patent: **12.08.2020**
(21) Application number: 15861282.0
(22) Date of filing: 13.11.2015
(51) Int. Cl.: A61M 1/14, A61M 1/16, A61K 31/19, A61K 33/00, A61M 1/36, A61K 33/14

(54) **DIALYSIS-FLUID SUPPLY SYSTEM**
DYALISEFLÜSSIGKEITZUFUHRSYSTEM
SYSTÈME D'ALIMENTATION DE FLUIDE DE DIALYSE

(30) Priority: 20.11.2014 JP 2014235458
(43) Date of publication of application: 27.09.2017
(73) Proprietor: Nikkiso Co., Ltd., Tokyo 150-6022 (JP)
(72) Inventor: FUJIWARA, Masato, Makinohara-shi, Shizuoka-ken 421-0496 (JP); MASUDA, Yoshimichi, Makinohara-shi, Shizuoka-ken 421-0496 (JP); NIMURA, Hiroshi, Makinohara-shi, Shizuoka-ken 421-0496 (JP); YOKOMICHI, Manabu, Higashimurayama-shi Tokyo 189-8520 (JP)
(74) Representative: Klunker IP Patentanwälte PartG mbB
(86) International application number: PCT/JP2015/082024
(87) International publication number: WO 2016/080318

(56) References cited:
- EP-A1- 0 469 487
- JP-A- H1 085 573
- JP-A- S6 365 874
- JP-A- 2004 041 742
- JP-A- 2004 041 742
- JP-A- 2007 117 396
- JP-B2- 3 685 887

## Description

### TECHNICAL FIELD

The present invention relates to a dialysis-fluid supply system according to the preamble of claim 1. The invention intends to generate dialysis fluid by mixing a diluent and at least two drugs and to output the dialysis fluid.

A supply system according to the preamble of claim 1 is known from JP 2004-041742. This document discloses to mix water and two solutions.

### BACKGROUND ART

In a conventionally known dialysis-fluid supply system, a plurality of drugs and a diluent (for example, water) are mixed together to generate and output dialysis fluid. Widely known examples of the dialysis-fluid supply system include a dialysis-fluid continuous supply system that generates dialysis fluid by continuously mixing a plurality of drugs and a diluent. The dialysis-fluid continuous supply system requires a measurement pump capable of accurately measuring the amounts of a diluent and a drug concentrated solution transferred for mixing. However, such a measurement pump capable of performing accurate measurement is typically expensive and requires frequent maintenance.

To avoid this problem, in a batch system, the drugs and the diluent in amounts necessary for achieving a desired concentration are collectively supplied to a tank to generate dialysis fluid, instead of continuously generating dialysis fluid. Patent Literatures 1 and 2 disclose such dialysis-fluid batch supply systems. In the batch system, drugs are measured in advance, or a measurement means is provided in the tank, which eliminates the need to provide an accurate measurement pump. This leads to further reduction in the price of the dialysis-fluid supply system and also in the amount of maintenance work.

### CITATION LIST

### PATENT LITERATURE

Patent Literature 1: National Publication of International Patent Application No. 2008-526375
Patent Literature 2: Japanese Patent Laid-Open Publication No. Hei 9-618
Patent Literature 3: USP 4,134,834

EP0469487A1 discloses an apparatus for dissolving an adjusting agent of a dialytic solution.

### SUMMARY OF INVENTION

### TECHNICAL PROBLEM

The invention is defined in the appended claims. Where in the following the word invention is used and/or features are presented as optional this should be interpreted in such a way that protection is sought only for the invention as claimed. However, the dialysis-fluid supply system disclosed in Patent Literature 1 is intended to employ lactic acid dialysis fluid used in peritoneal dialysis, but not bicarbonate dialysis fluid widely used in blood dialysis treatment nowadays. The dialysis-fluid supply system disclosed in Patent Literature 2 is intended to employ bicarbonate dialysis fluid, but can be used for dialysis treatment in a relatively short time (for example, two hours). Specifically, bicarbonate dialysis fluid is generated by mixing a diluent and two kinds of drugs called drugs A and B. However, when a certain time (for example, two hours) has elapsed after the mixing of the drugs A and B, a deposit is generated in this bicarbonate dialysis fluid, and the bicarbonate dialysis fluid can no longer be used in blood dialysis treatment. Thus, in the technology of Patent Literature 2, in which dialysis fluid is generated in one tank, the dialysis fluid can be output only in an amount that can be used up within this certain time. However, a continuous dialysis treatment in a longer time (for example, six hours) is required depending on the condition and lifestyle of a patient, and such a requirement cannot be sufficiently met by the technology of Patent Literature 2.

Patent Literature 3 discloses a system including two tanks. In this system, while one of the tanks outputs dialysis fluid, the other tank generates dialysis fluid, and when the one tank runs out of the dialysis fluid, the other tank outputs the dialysis fluid while the one tank generates dialysis fluid. However, the dialysis-fluid supply system of Patent Literature 3 is not intended to employ bicarbonate dialysis fluid. Moreover, the dialysis-fluid supply system of Patent Literature 3 includes a measurement pump to measure a diluent, resulting in an expensive system that requires frequent maintenance work. In addition, in the technology of Patent Literature 3, switching is performed between a tank that generates dialysis fluid and a tank that outputs the dialysis fluid. Thus, in the technology of Patent Literature 3, the two tanks each need to function as the dialysis-fluid generation tank and the dialysis-fluid storage tank. With this configuration, the technology of Patent Literature 3 requires, for the two tanks, two sets of valves, measurement pumps, drug supply mechanisms, which is wasteful.

It is an advantage of the present invention to provide a dialysis-fluid supply system that generates and outputs dialysis fluid obtained by mixing a plurality of drugs, and is inexpensive and capable of outputting a large amount of the dialysis fluid.

### SOLUTION TO PROBLEM

A dialysis-fluid supply system according to the present invention is a dialysis-fluid supply system that generates dialysis fluid by mixing a diluent and at least two drugs, and outputs the dialysis fluid. The dialysis-fluid supply system includes the features of claim 1.

In a preferable aspect, the control unit may generate dialysis fluid in the mixing tank while outputting dialysis fluid from the storage tank to a dialysis device. In another preferable aspect, the mixing tank may include a weight sensor that measures the weight of supplied fluid or a level sensor that measures the level of supplied fluid, and the control unit may monitor the supply amount of each drug or the diluent to the mixing tank based on a result of detection by the weight sensor or the level sensor.

In another preferable aspect, the dialysis-fluid supply system may further include a circulation mechanism that internally and externally circulates fluid inside the mixing tank to perform agitation. In another preferable aspect, the capacity of the storage tank may be larger than the capacity of the mixing tank. In another preferable aspect, the control unit may execute a cleaning process of cleaning the mixing tank and the storage tank by causing cleaning fluid to flow through the mixing tank and the storage tank in this order.

### ADVANTAGEOUS EFFECTS OF INVENTION

In a dialysis-fluid supply system according to the present invention, dialysis fluid is generated by a mixing tank and stored in a storage tank for outputting, which allows continuous generation and outputting of the dialysis fluid for a long time without a measurement pump. Accordingly, the dialysis-fluid supply system is inexpensive and is capable of outputting a large amount of dialysis fluid compared to a conventional dialysis-fluid supply system.

### BRIEF DESCRIPTION OF DRAWINGS

FIG. 1 is a diagram illustrating the configuration of a dialysis system according to an embodiment of the present invention.
FIG. 2 is a timing chart illustrating timing of opening and closing of valves in a dialysis-fluid supply system.

### DESCRIPTION OF EMBODIMENT

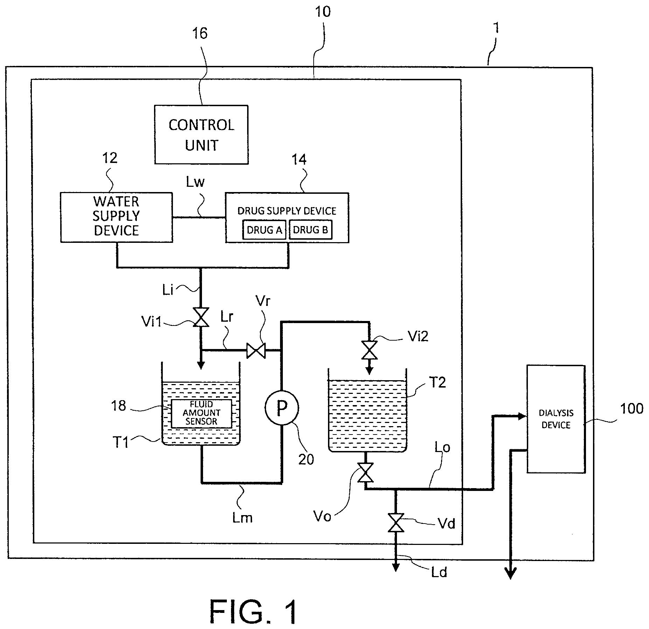

The following describes a dialysis system 1 according to an embodiment of the present invention with reference to the accompanying drawings. FIG. 1 illustrates the configuration of a dialysis-fluid supply system 10 according to the embodiment of the present invention. The dialysis system 1 illustrated in FIG. 1 includes the dialysis-fluid supply system 10 and a dialysis device 100 connected with the dialysis-fluid supply system 10. The dialysis device 100 cleans blood by circulating the blood out of the body of a patient through: a blood circulating system including a dialyzer that cleans blood by allowing blood and dialysis fluid to come in to contact with each other through a semipermeable membrane, an arterial blood circuit that takes in blood from the patient and inputs the blood to the dialyzer, and a venous blood circuit that returns, to the patient, blood output from the dialyzer; a dialysis-fluid supplying and discharging system including lines that supply and discharge the dialysis fluid to and from the dialyzer; and a blood pump provided to the arterial blood circuit. The dialysis-fluid supply system 10 generates dialysis fluid by mixing a diluent and a plurality of drugs, and supplies the generated dialysis fluid to the dialysis device 100.

The dialysis-fluid supply system 10 has a relatively small-sized configuration suitable for medical treatment of a small number (for example, one) of patients, and is expected to be used in a relatively small-sized facility such as a hospital or a home, but not in a dialysis clinic. However, the capacities of tanks T1 and T2 to be described later can be changed to provide a configuration suitable for simultaneous medical treatment of a large number of patients and use at a large-sized facility. The dialysis-fluid supply system 10 and the dialysis device 100 are preferably integrated to achieve reduction in the size of the entire system, but may be separated.

The dialysis-fluid supply system 10 mainly includes the mixing tank T1 and the storage tank T2, a supply mechanism that supplies a drug and a diluent to the mixing tank T1, a transfer mechanism that transfers dialysis fluid generated by the mixing tank T1 to the storage tank T2, an output mechanism that outputs dialysis fluid stored in the storage tank T2 to the dialysis device 100, and a control unit 16 that controls driving of various mechanisms.

The mixing tank T1 is a container for generating dialysis fluid by mixing and diluting water and a drug. The mixing tank T1 includes a fluid amount sensor 18 that detects the amount of supplied fluid, such as a level switch that detects a fluid level, or a load sensor that detects the weight of supplied fluid. The mixing tank T1 desirably includes a concentration sensor (not illustrated) that detects the concentration of the dialysis fluid generated in the mixing tank T1.

The storage tank T2 is a container for storing the dialysis fluid generated by the mixing tank T1.The dialysis fluid generated by the mixing tank T1 is transferred to the storage tank T2 by a transfer mechanism to be described later. The dialysis fluid stored in the storage tank T2 is output to the dialysis device 100 at a constant speed by the output mechanism. The storage tank T2 desirably includes the fluid amount sensor 18 that detects the amount of stored fluid to detect overflow of dialysis fluid from the storage tank T2 and the amount of remaining dialysis fluid. However, the fluid amount sensor 18 may be omitted from the storage tank T2 when it is possible to accurately control timing of generation and use of dialysis fluid, as described later.

The capacities of the mixing tank T1 and the storage tank T2 are determined in accordance with a use limit time duration of dialysis fluid. In the present embodiment, the dialysis fluid is bicarbonate dialysis fluid. The bicarbonate dialysis fluid is, as is well known, obtained by mixing and diluting two kinds of drugs, which are a drug A and a drug B. The drug A includes an electrolyte component (for example, sodium chloride, potassium chloride, calcium chloride, magnesium chloride, or sodium acetate), a pH adjuster (for example, acetic acid), and sugar (for example, glucose). The drug B includes sodium bicarbonate.

The concentration of the bicarbonate dialysis fluid changes due to generation of a deposit when a certain time elapses after mixing of the drugs A and B, and thus a time duration in which the bicarbonate dialysis fluid is usable after its generation, in other words, the use limit time duration, is set. The use limit time duration differs slightly between kinds of product, but is typically two hours approximately. In the present embodiment, the mixing tank T1 has a capacity equal to or less than the amount of dialysis fluid generated and used in the use limit time duration. For example, when the use limit time duration is two hours and the flow rate of dialysis fluid used in dialysis treatment is 500 mL/min, the mixing tank T1 desirably has a capacity equal to or less than the amount of dialysis fluid used in the two-hour dialysis treatment, which is 500 mL x 120min = 60 L. Since consideration needs to be given to such a problem that a time duration until a deposit is generated since the drugs A and B are mixed changes with solution temperature and to a time duration for which the dialysis fluid is stored after production, the use limit time duration is preferably set to be one hour to reliably prevent a deposit. In this case, when the flow rate of dialysis fluid is 500 mL/min, the mixing tank T1 desirably has a capacity equal to or less than 500 mL x 60 min = 30 L. Note that, of course, the capacity of the mixing tank T1 may be changed in accordance with the flow rate of dialysis fluid. Thus, for example, when the flow rate of dialysis fluid used in a dialysis treatment is 250 mL/min, the mixing tank T1 may have a maximum capacity equal to or less than 250 mL x 60 min = 15 L. Production of dialysis fluid needs to take at least 15 minutes to prevent insufficient dissolution of the drugs, and thus the mixing tank T1 desirably has a capacity equal to or more than the amount of dialysis fluid used in a dialysis treatment for 15 minutes. Specifically, the mixing tank T1 desirably has a capacity equal to or more than 7.5 L when the flow rate of dialysis fluid is 500 mL/min, or a capacity equal to or more than 3.75 L when the flow rate of dialysis fluid is the 250 mL/min.

The storage tank T2 desirably has a capacity larger than the capacity of the mixing tank T1 to prevent overflow of dialysis fluid from the storage tank T2. In order to prevent discontinuity of dialysis fluid output from the storage tank T2 in a dialysis treatment, the dialysis fluid needs to be transferred from the mixing tank T1 to the storage tank T2 before the storage tank T2 completely runs out of the dialysis fluid, in other words, while some dialysis fluid remains in the storage tank T2. Thus, to prevent the overflow of dialysis fluid from the storage tank T2, the storage tank T2 needs to have a capacity equal to or more than the sum of the capacity of the mixing tank T1 and the amount of remaining dialysis fluid.

The supply mechanism includes a water supply device 12 that supplies water as the diluent, a drug supply device 14 that supplies the drugs A and B, an input line Li connected with these devices, and a first input valve Vi1 provided in the input line Li. The water supply device 12 may have, but is not particularly limited to, any configuration capable of supplying highly pure water. Thus, the water supply device 12 may be, for example, an RO device that generates highly pure RO water by removing impurities from water through a reverse osmosis membrane (RO membrane), or a water treatment device that generates highly pure water through ion exchange resin and an ultrafiltration membrane (UF membrane). The water supply device 12 supplies water to the mixing tank T1 and the drug supply device 14 through the lines Lw and Li.

The drug supply device 14 supplies drugs for dialysis fluid to the mixing tank T1. In the present embodiment, since the dialysis fluid is bicarbonate dialysis fluid, the supplied drugs are the drugs A and B. The drugs A and B are set to the drug supply device 14 in advance, and supplied to the mixing tank T1 together with water. The drugs A and B set in the drug supply device 14 are desirably individually packaged in advance in an amount necessary for each generation of the dialysis fluid. When the dialysis-fluid supply system 10 includes a mechanism for measuring the drugs A and B, however, the drugs A and B do not need to be measured and packaged in advance.

The drugs A and B set in the drug supply device 14 may be provided in powder form or tablet form, or may be provided as concentrated solution dissolved with a small amount of water. Alternatively, one of the drugs A and B set in the drug supply device 14 may be in liquid form, and the other may be in solid form (powder form or tablet form). The drug supply device 14 may supply the drugs A and B set in powder form or tablet form directly to the mixing tank T1, or may supply the drugs A and B to the mixing tank T1 as a concentrated solution dissolved with a small amount of water. The water supply device 12 supplies water to the drug supply device 14 through the line Lw, and a concentrated solution obtained by dissolving the drugs A and B with water is supplied to the mixing tank T1.In any case, the drug supply device 14 only needs to supply a necessary amount of the drugs of the dialysis fluid to the mixing tank T1. In the present embodiment, the bicarbonate dialysis fluid is exemplarily described, but the dialysis fluid may be any other kind of dialysis fluid generated by mixing and diluting a plurality of drugs.

The water from the water supply device 12 and the drugs from the drug supply device 14 are output to the input line Li. The input line Li is a line that is connected with the mixing tank T1 and through which the drugs and the water are supplied to the mixing tank T1. The input line Li is provided with the first input valve Vi1, opening and closing of which is driven by the control unit 16.

The transfer mechanism transfers dialysis fluid generated by the mixing tank T1 to the storage tank T2, and includes a link line Lm, a second input valve Vi2, and a transfer pump 20. The link line Lm is a line that couples the mixing tank T1 and the storage tank T2 with each other and in which the transfer pump 20 and the second input valve Vi2 are provided. The control unit 16 drives the transfer pump 20 to transfer the dialysis fluid from the mixing tank T1 to the storage tank T2. During the transfer, the second input valve Vi2 is opened.

The link line Lm is connected with a circulation line Lr. The circulation line Lr is a line that couples the link line Lm and the input line Li with each other and in which a circulation valve Vr is provided. The fluid inside the mixing tank T1 is internally and externally circulated through the circulation line Lr to perform agitation of the drugs and the water. Specifically, the agitation of the drugs and the water is performed by driving the transfer pump 20 while the circulation valve Vr is opened and the first and second input valves Vi1 and Vi2 are closed. Accordingly, the fluid inside the mixing tank T1 returns to the mixing tank T1 through the link line Lm, the circulation line Lr, and the input line Li, and this circulation agitates the fluid inside the mixing tank T1. This can prevent insufficient dissolution and remaining dialysis drugs.

The output mechanism outputs dialysis fluid stored in the storage tank T2 to the dialysis device 100, and includes an output line Lo and an output valve Vo. The output line Lo is a line that couples the storage tank T2 and the dialysis device 100 with each other and in which the output valve Vo is provided. While the dialysis fluid is output, in other words, while a dialysis treatment is performed, the output valve Vo is kept opened. The output line Lo is connected with a discard line Ld. The discard line Ld is a line that couples the output line Lo and a drain with each other and in which a discard valve Vd is provided. Any dialysis fluid remaining after the dialysis treatment has ended and fluid (cleaning fluid and rinsing water) used to clean the tanks T1 and T2 are discarded through the discard line Ld.

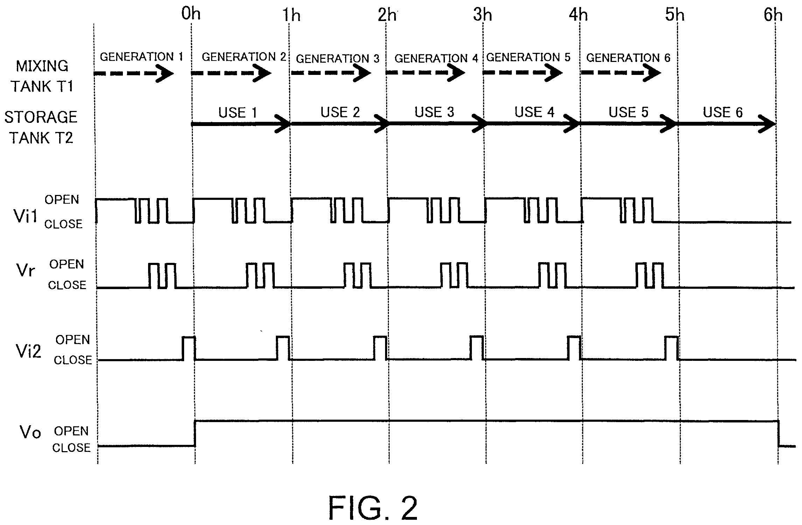

The control unit 16 controls driving of, for example, the water supply device 12, the drug supply device 14, and the valves Vi1, Vi2, Vo, and Vr described above. In the present embodiment, the control unit 16 switches opening and closing of the valves to generate dialysis fluid in the mixing tank T1 while outputting dialysis fluid from the storage tank T2, and transfer the dialysis fluid in the mixing tank T1 to the storage tank T2 when the amount of dialysis fluid remaining in the storage tank T2 becomes low. FIG. 2 is a timing chart illustrating timing of the opening and closing of the valves Vi1, Vi2, Vo, and Vr when a dialysis treatment is continuously performed with the dialysis-fluid supply system 10 for six hours.

When a dialysis treatment is started, first, dialysis fluid is generated in the mixing tank T1. The generation of the dialysis fluid is achieved by sequentially performing supply of water to the mixing tank T1, supply of the drug B to the mixing tank T1, agitation, supply of the drug A to the mixing tank T1, and agitation. Specifically, first, the first input valve Vi1 is opened to supply a certain amount of water into the mixing tank T1. This supply amount of water is controlled based on a result of detection by the fluid amount sensor 18 provided to the mixing tank T1. Subsequently, while the first input valve Vi1 is opened, the drug B is supplied. Simultaneously, water is supplied together with the drug B, and this supply amount of water is controlled based on a result of detection by the fluid amount sensor 18. After the drug B is supplied, the transfer pump 20 is driven while the first input valve Vi1 is closed and the circulation valve Vr is opened, so as to circulate (agitate) the fluid inside the tank T1. Once the agitation is completed, the concentration of the fluid inside the mixing tank T1 is detected by the concentration sensor (not illustrated). If the detected concentration is within a predetermined reference range, the first input valve Vi1 is then opened and the circulation valve Vr is closed to supply the drug A to the mixing tank T1. The amount of water supplied together with the drug A is controlled based on a result of detection by the fluid amount sensor 18. After the drug A is supplied, the transfer pump 20 is driven again while the first input valve Vi1 is closed and the circulation valve Vr is opened, so as to circulate (agitate) the fluid inside the tank T1. Then, the concentration of the fluid inside the mixing tank T1 is detected by the concentration sensor again, and if the detected concentration has no problem, the generation of dialysis fluid is completed.

Once the dialysis fluid is generated in the mixing tank T1, the second input valve Vi2 is opened and the transfer pump 20 is driven to transfer the dialysis fluid from the mixing tank T1 to the storage tank T2. Once the dialysis fluid is transferred to the storage tank T2, a dialysis treatment is started. Specifically, the output valve Vo is opened to output the dialysis fluid stored in the storage tank T2 to the dialysis device 100. Until 6h, at which the dialysis treatment is ended, the output valve Vo is kept opened to continuously transfer a certain amount of dialysis fluid to the dialysis device 100. As described above, the mixing tank T1 generates dialysis fluid in an amount used in a one-hour dialysis treatment. Thus, dialysis fluid (Use N) output between (N-1) h to Nh (N is an integer of one to six) in the storage tank T2 is dialysis fluid (Generation N) generated at the N-th time in the mixing tank T1.

After the generated dialysis fluid is transferred to the storage tank T2, the mixing tank T1 starts the second generation of dialysis fluid. Once the generation is completed, the generated dialysis fluid is transferred from the mixing tank T1 to the storage tank T2 if the remaining amount of the dialysis fluid inside the storage tank T2 becomes lower than a predetermined threshold. Then, the same processing is repeated to perform the generation and transfer of dialysis fluid a total of six times.

Before generation and outputting of dialysis fluid, the mixing tank T1 and the storage tank T2 are cleaned. At the time of cleaning, first, cleaning fluid is supplied and stored in the mixing tank T1 to clean the mixing tank T1. Subsequently, the cleaning fluid in the mixing tank T1 is transferred to the storage tank T2 and stored therein. Once the storage tank T2 is cleaned, the stored cleaning fluid is discharged to the drain. Subsequently, rinsing water is sequentially transferred to the mixing tank T1 and the storage tank T2 and then discharged to the drain.

As described above, in the present embodiment, the two tanks T1 and T2 are provided, the tank T1 generating dialysis fluid, and the tank T2 outputting the dialysis fluid. This configuration is employed for the following reasons.

In most conventional dialysis-fluid supply systems, dialysis fluid is continuously generated and continuously supplied. Specifically, in the conventional dialysis-fluid supply system, a concentrated solution of the drugs A and B, which is obtained in advance, and water, are accurately measured by a measurement pump before flowing into a line, and then mixed together in the line to generate dialysis fluid at a desired concentration. The measurement pump provided to this dialysis-fluid continuous supply system needs to be able to accurately measure the amount of fluid and transfer the fluid. However, typically, such a measurement pump is extremely expensive and needs frequent maintenance, and thus is difficult to use at a small-sized facility.

To solve this difficulty, a dialysis-fluid batch supply system has been proposed in which certain amounts of drugs and water are supplied to a tank to generate dialysis fluid. In such a dialysis-fluid batch supply system, when the tank includes a fluid amount sensor, no measurement pump is needed, thereby achieving reduction in the price of the entire system and also in maintenance work. However, only one tank that generates dialysis fluid is provided in the conventional dialysis-fluid batch supply system. Thus, the system could not generate a large amount of dialysis fluid.

As described above, the use limit time duration is set for the bicarbonate dialysis fluid. When the bicarbonate dialysis fluid is generated in one tank in excess of the use limit time duration, some of the dialysis fluid that has not been used in the use limit time duration is discarded. Thus, in a configuration in which dialysis fluid is generated in one tank only, part of the dialysis fluid in excess of an amount used in medical treatment in the use limit time duration (two hours approximately) cannot be output. Accordingly, when a dialysis-fluid supply system provided with one tank only is employed, a dialysis treatment can be continuously performed over a time duration less than the use limit time duration.

However, a dialysis treatment is continuously performed for a longer time duration depending on the condition and lifestyle of a patient. In particular, it has been desired to reduce the frequency of dialysis treatment by performing the dialysis treatment for a long time (for example, six hours) while the patient is sleeping. However, the conventional dialysis-fluid supply system provided with only one tank could not sufficiently meet such a demand by patients.

To solve this problem, in the present embodiment, as described above, the two tanks T1 and T2 are provided, the mixing tank T1 generating dialysis fluid, and the storage tank T2 storing the generated dialysis fluid. This configuration allows continuous outputting of the dialysis fluid generated within the use limit time, without discontinuity. Accordingly, the freedom of medical treatment time can be increased in accordance with the condition and lifestyle of a patient. In the present embodiment, since the mixing tank T1 includes the fluid amount sensor 18, a pump that transfers, for example, water does not need to have a measurement function, which leads to reduction in the price of the entire system and also in the amount of maintenance work.

Patent Literature 3 discloses a technology in which two tanks are provided such that a tank that generates dialysis fluid and a tank that outputs the dialysis fluid are sequentially switched. In this technology too, dialysis fluid generated within a use limit time can be continuously output without discontinuity. In the technology of Patent Literature 3, however, each tank functions as the dialysis-fluid generation tank and the dialysis-fluid outputting tank. Accordingly, the technology of Patent Literature 3 requires, for the two tanks, two sets of various valves and sensors necessary for functioning as the dialysis-fluid generation tank and various valves and sensors necessary for functioning as the dialysis-fluid storage tank. In the present embodiment, the tank T1, as one of the tanks, has a dialysis-fluid generation function only, and the other tank T2 has a dialysis-fluid outputs function only. This configuration only requires, for the one tank, one set of various valves and sensors necessary for functioning as the dialysis-fluid generation tank, and only requires, for the other tank, one set of various valves and sensors necessary for functioning as the dialysis-fluid storage tank. Accordingly, the present embodiment can achieve further reduction in cost of the entire system with a simpler configuration.

The above-described configuration is merely exemplary. Modifications of the configuration are possible as appropriate to achieve a configuration including the mixing tank T1, which generates dialysis fluid, and the storage tank T2, which stores therein and outputs the dialysis fluid generated by the mixing tank. For example, in the present embodiment, the drugs (drugs A and B) are automatically supplied by the drug supply device 14, but may be manually supplied. In the present embodiment, the transfer pump 20 is provided to transfer dialysis fluid from the mixing tank T1 to the storage tank T2, but a height difference between the tanks may be exploited to transfer the dialysis fluid without the pump. In the above-described embodiment, fluid inside the mixing tank T1 is agitated by being internally and externally circulated. However, the agitation is not limited to this method but may be performed by another method. For example, a bladed wheel for agitation may be provided in each tank to perform the agitation.

### REFERENCE SIGNS LIST

- 1: Dialysis system
- 10: Dialysis-fluid supply system
- 12: Water supply device
- 14: Drug supply device
- 16: Control unit
- 18: Fluid amount sensor
- 20: Transfer pump
- 100: Dialysis device
- Ld: Discard line
- Li: Input line
- Lm: Link line
- Lo: Output line
- Lr: Circulation line
- T1: Mixing tank
- T2: Storage tank
- Vd: Discard valve
- Vi1: First input valve
- Vi2: Second input valve
- Vo: Output valve
- Vr: Circulation valve

## Claims

1. A dialysis-fluid supply system (10) that generates dialysis fluid by mixing a diluent and at least two drugs and outputs the dialysis fluid, the system comprising:
a mixing tank (T1) that generates dialysis fluid by mixing the drugs and the diluent;
a storage tank (T2) that stores therein the dialysis fluid generated by the mixing tank and outputs the dialysis fluid;
a transfer mechanism that transfers the dialysis fluid generated by the mixing tank to the storage tank;
an output mechanism that outputs the dialysis fluid stored in the storage tank to a dialysis device;
a concentration sensor that detects a concentration of the dialysis fluid within the mixing tank; and
a control unit (16) that controls driving of the transfer mechanism and the output mechanism, wherein
the dialysis-fluid supply system is an individual dialysis-fluid supply system used for treatment of one patient;
the at least two drugs are provided in a powder form;
the at least two drugs are individually packaged in an amount necessary for each generation of the dialysis solution;
the dialysis-fluid supply system further comprises a drug supply device that supplies the at least two drugs directly to the mixing tank;
the dialysis fluid is obtained by mixing and diluting a drug A and a drug B;
the drug A comprises an electrolyte component, a pH adjuster, and sugar;
the drug B comprises sodium bicarbonate; and
the control unit controls the drug supply device to execute a process of supplying water together with the drug B to the mixing tank and thereafter detecting the concentration of the drug B within the mixing tank by the concentration sensor and a process of supplying water together with the drug A to the mixing tank and thereafter detecting the concentration of the drug A within the mixing tank by the concentration sensor.

2. The dialysis-fluid supply system according to claim 1, wherein the control unit causes the mixing tank to generate dialysis fluid while causing the storage tank to output dialysis fluid to a dialysis device.

3. The dialysis-fluid supply system according to claim 1 or 2, wherein
the mixing tank includes a weight sensor that measures the weight of supplied fluid or a level sensor that measures the level of supplied fluid, and
the control unit monitors the supply amount of each drug or the diluent to the mixing tank based on a result of detection by the weight sensor or the level sensor.

4. The dialysis-fluid supply system according to any one of claims 1 to 3, further comprising a circulation mechanism that internally and externally circulates fluid inside the mixing tank to perform agitation.

5. The dialysis-fluid supply system according to any one of claims 1 to 4, wherein the capacity of the storage tank is larger than the capacity of the mixing tank.

6. The dialysis-fluid supply system according to any one of claims 1 to 5, wherein the control unit executes a cleaning process of cleaning the mixing tank and the storage tank by causing cleaning fluid to flow through the mixing tank and the storage tank in this order.

## Patentansprüche

1. Dialysefluid-Zuführsystem (10), das Dialysefluid erzeugt, indem es ein Verdünnungsmittel und mindestens zwei Medikamente mischt und das Dialysefluid abgibt, umfassend:
einen Mischgang (T1), der Dialysefluid durch Mischen der Medikamente und des Verdünnungsmittel erzeugt;
einen Speichertank (T2), der in sich das von dem Mischtank erzeugte Dialysefluid speichert und das Dialysefluid abgibt;
einen Transfermechanismus, der das von dem Mischtank erzeugte Dialysefluid zu dem Speichertank transferiert;
einen Abgabemechanismus, der das in dem Speichertank gespeicherte Dialysefluid an ein Dialysegerät abgibt;
einen Konzentrationssensor, der eine Konzentration des Dialysefluids in dem Mischtank detektiert; und
eine Steuereinheit (16), die den Antrieb des Transfermechanismus' und des Abgabemechanismus steuert, wobei
das Dialysefluid-Zuführsystem ein individuelles Dialysefluid-Zuführsystem ist, welches für die Behandlung eines Patienten verwendet wird;
die mindestens zwei Medikamente in Pulverform vorgesehen sind;
die mindestens zwei Medikamente individuell in einer Menge abgepackt sind, die für jeweiliges Erzeugen der Dialyselösung notwendig ist;
das Dialysefluid-Zuführsystem weiterhin eine Medikamentenzuführeinrichtung aufweist, die die mindestens zwei Medikamente direkt dem Mischtank zuführt;
das Dialysefluid erhalten wird durch Mischen und Verdünnen eines Medikaments A und eines Medikaments B;
das Medikament A eine Elektrolytkomponente, einen pH-Wert-Einsteller und Zucker aufweist;
das Medikament B Natriumbicarbonat aufweist; und
die Steuereinheit die Medikamentenzuführeinrichtung steuert zum Ausführen eines Prozesses des Zuführens von Wasser zusammen mit dem Medikament B zu dem Mischtank und zum anschließenden Detektieren der Konzentration des Medikaments B innerhalb des Mischtanks durch den Konzentrationssensor, und einen Prozess steuert zum Zuführen von Wasser zusammen mit dem Medikament A zu dem Mischtank und zum anschließenden Detektieren der Konzentration des Medikaments A innerhalb des Mischtanks durch den Konzentrationssensor.

2. Dialysefluid-Zuführsystem nach Anspruch 1, bei dem die Steuereinheit den Mischtank veranlasst, Dialysefluid zu erzeugen, während der Speichertank veranlasst wird, Dialysefluid an das Dialysegerät abzugeben.

3. Dialysefluid-Zuführsystem nach Anspruch 1 oder 2, bei dem
der Mischtank einen Gewichtssensor enthält, der das Gewicht von zugeführtem Fluid misst, oder einen Pegelsensor enthält, der den Pegel des zugeführten Fluids misst, und
die Steuereinheit die Zuführmenge jedes Medikaments oder des Verdünnungsmittels zu dem Mischtank basierend auf dem Nachweis durch den Gewichtssensor oder den Pegelsensor überwacht.

4. Dialysefluid-Zuführsystem nach einem der Ansprüche 1 bis 3, weiterhin umfassend einen Zirkulationsmechanismus, der von innen oder von außen das Fluid im Inneren des Mischtanks umwälzt, um ein Umrühren zu erreichen.

5. Dialysefluid-Zuführsystem nach einem der Ansprüche 1 bis 4, bei dem die Kapazität des Speichertanks größer ist als die Kapazität des Mischtanks.

6. Dialysefluid-Zuführsystem nach einem der Ansprüche 1 bis 5, bei dem die Steuereinheit einen Reinigungsprozess zum Reinigen des Mischtanks und des Speichertanks ausführt, indem sie veranlasst, dass Reinigungsfluid durch den Mischtank und den Speichertank in dieser Reihenfolge strömt.

## Revendications

1. Système d'alimentation en liquide de dialyse (10), lequel génère un liquide de dialyse en mélangeant un diluant et au moins deux médicaments, et produit le liquide de dialyse, le système comprenant :
un réservoir de mélange (T1), lequel génère un liquide de dialyse en mélangeant les médicaments et le diluant ;
un réservoir de stockage (T2), lequel stocke à l'intérieur le liquide de dialyse généré par le réservoir de mélange et produit le liquide de dialyse ;
un mécanisme de transfert, lequel transfère le liquide de dialyse stocké par le réservoir de mélange vers le réservoir de stockage ;
un mécanisme de sortie, lequel fait sortir le liquide de dialyse stocké dans le réservoir de stockage vers un dispositif de dialyse ;
un capteur de concentration, lequel détecte une concentration du liquide de dialyse dans le réservoir de mélange, et
une unité de commande (16), laquelle commande l'entraînement du mécanisme de transfert et le mécanisme de sortie, dans lequel
le système d'alimentation en liquide de dialyse est un système d'alimentation en liquide de dialyse individuel utilisé pour le traitement d'un patient ;
les au moins deux médicaments sont prévus sous une forme pulvérulente ;
les au moins deux médicaments sont emballés individuellement en une quantité nécessaire pour chaque génération de la solution de dialyse ;
le système d'alimentation en liquide de dialyse comprend en outre un dispositif d'alimentation en médicament, lequel fournit les au moins deux médicaments directement vers le réservoir de mélange ;
le liquide de dialyse est obtenu en mélangeant et en diluant un médicament A et un médicament B ;
le médicament A comprend un composant électrolyte, un agent d'ajustement de pH, et du sucre ;
le médicament B comprend du bicarbonate de sodium, et
l'unité de commande commande le dispositif d'alimentation en médicament afin d'exécuter un processus d'alimentation en eau conjointement au médicament B vers le réservoir de mélange, puis de détection de la concentration en médicament B dans le réservoir de mélange par le capteur de concentration, et un processus d'alimentation en eau conjointement au médicament A vers le réservoir de mélange, puis de détection de la concentration en médicament A dans le réservoir de mélange par le capteur de concentration.

2. Système d'alimentation en liquide de dialyse selon la revendication 1, dans lequel l'unité de commande fait en sorte que le réservoir de mélange génère un liquide de dialyse tout en faisant en sorte que le réservoir de stockage produise le liquide de dialyse vers un dispositif de dialyse.

3. Système d'alimentation en liquide de dialyse selon la revendication 1 ou 2, dans lequel
le réservoir de mélange inclut un capteur de poids, lequel mesure le poids de liquide alimenté ou un capteur de niveau, lequel mesure le niveau de liquide alimenté, et
l'unité de commande surveille la quantité d'alimentation de chaque médicament ou du diluant vers le réservoir de mélange, sur la base d'un résultat de détection par le capteur de poids ou le capteur de niveau.

4. Système d'alimentation en liquide de dialyse selon l'une quelconque des revendications 1 à 3, comprenant en outre un mécanisme de circulation, lequel fait circuler de manière interne et externe un fluide dans le réservoir de mélange afin de réaliser une agitation.

5. Système d'alimentation en liquide de dialyse selon l'une quelconque des revendications 1 à 4, dans lequel la capacité du réservoir de stockage est supérieure à la capacité du réservoir de mélange.

6. Système d'alimentation en liquide de dialyse selon l'une quelconque des revendications 1 à 5, dans lequel l'unité de commande exécute un processus de nettoyage du réservoir de mélange et du réservoir de stockage en faisant en sorte que le fluide de nettoyage s'écoule à travers le réservoir de mélange et le réservoir de stockage dans cet ordre.
